# EUROPEAN PATENT SPECIFICATION

(11) **EP 0 584 214 B1**
(45) Date of publication and mention of the grant of the patent: **28.07.2004**
(21) Application number: 92911887.5
(22) Date of filing: 05.05.1992
(51) Int. Cl.: C12N 15/85, C12N 15/81, C12P 21/06, C12N 15/53, C12N 1/19, C12N 5/10, C12N 9/64, C07K 14/59, C07K 14/535

(54) **GENE MANIPULATION AND EXPRESSION USING GENOMIC ELEMENTS**
GENE MANIPULATION UND EXPRESSION MITTELS GENOMISCHER ELEMENTE
MANIPULATION ET EXPRESSION DE GENES A L'AIDE D'ELEMENTS DE GENOME

(30) Priority: 06.05.1991 US 696216
(43) Date of publication of application: 02.03.1994
(73) Proprietor: CELL GENESYS, INC., Foster City, CA 94404 (US)
(72) Inventor: SHERWIN, Stephen, San Francisco, CA 94118 (US); KLAPHOLZ, Sue, Stanford, CA 94305 (US); SKOULTCHI, Arthur, Larchmont, NY 10538 (US)
(74) Representative: Hallybone, Huw George
(86) International application number: PCT/US1992/003686
(87) International publication number: WO 1992/019255

(56) References cited:
- WO-A-91/06667
- WO-A-91/09955
- US-A- 4 656 134
- US-A- 4 740 461
- J. SAMBROOK ET AL 'Molecular Cloning' 1989 , COLD SPRING HARBOR LABORATORY PRESS * page 9.5 - page 9.6 * * page 16.5, paragraph 2 - page 16.6, paragraph 3 * * page 16.9, last paragraph - page 16.10, paragraph 3 *
- PROCEEDINGS OF THE NATIONAL ACADEMY OF SCIENCES OF USA., vol.84, October 1987, WASHINGTON US pages 6820 - 6824 K-Y SONG ET AL 'Accurate modification of a chromosomal plasmid by homologous recombination in human cells'
- GENE, vol.29, 1984 pages 231 - 241 O. RAIBAUD ET AL 'A technique for integrating any DNA fragment into the chromosome of Escherichia coli'
- Proceedings National Academy of Sciences, Volume 86, issued August 1989, TRAVER et al., "Roped Screening of a human genomic library in yeast artificial chromosomes for single copy". Sequences, pages 5898-5902, entire document.
- Cell, Volume 51, issued 06 November 1987, THOMAS et al., "Site-Directed Mutagenesis by Gene Targeting or Mouse Embryo-Derived Stem Cells", pages 503-512, entire document.
- Science, Volume 244, issued 16 June 1989, BROWNSTEIN et al., "Isolation of Single-copy human genes from a library of yeast Artificial Chromosome Clones", pages 1348-1351, entire document.
- Science, Volume 240, issued 10 June 1988, BOTSTEIN et al., "Yeast: An experimental Organism for Modern Porology", pages 1439-1443, entire document.
- Proceedings National Academy of Sciences, Volume 87, issued November 1990, SMITH et al., "Amplification of large artificial chromosomes", pages 8242-8246, entire document.
- GANSBACHER ET AL: 'Retroviral Vector-mediated gamma-Interferon Gene Transfer into Tumour Cells Generates Potent and Long Lasting Antitumour Immunity' CANCER RESEARCH vol. 50, 15 December 1990, pages 7820 - 7825

## Description

### INTRODUCTION

### Technical Field

The field of this invention concerns the manipulation and expression of mammalian genes.

### Background

with the development of genetic engineering over the last two decades, including restriction enzymes, reverse transcriptase, cloning, polymerase chain reaction, sequencing, and monoclonal antibodies, there has been an extraordinary increase in the ability to isolate, identify and manipulate nucleic acid sequences. As a result of these capabilities, numerous genes and their transcriptional control elements have been identified and manipulated. The genes have been used for producing large amounts of a desired protein in heterologous hosts (bacterial and eukaryotic host cell systems).

In many cases, the process of obtaining coding sequences and eliciting their expression has been a long and arduous one. The identification of the coding sequence, either cDNA or genomic DNA, has frequently involved the construction of libraries, identification of fragments of the open reading frame, examining the flanking sequence, and the like. In mammalian genes where introns are frequently encountered, in many instances, the coding region has been only a small fraction of the total nucleic acid associated with the gene. In other cases, pseudogenes or multi-membered gene families have obscured the ability to isolate a particular gene of interest. Nevertheless, as techniques have improved, there has been a continuous parade of successful identifications and isolation of genes of interest.

For many reasons, it may be desirable to manipulate the coding region or the transcriptional regulatory regions without isolating the coding region or cloning the coding region on a fragment where the coding region is the primary sequence. These reasons may include ease of manipulation, development of different pathways for expression, or the like.

Also, in many situations, one is primarily interested in a source of the protein product. The cell type in the body which produces the product is frequently an inadequate source. There is, therefore, significant interest in developing alternative techniques for producing proteins of interest in culture, with cells which provide for economic and efficient production of the desired protein and, when possible, appropriate processing of the protein product.

### Relevant Literature

Mansour *et al.,* Nature, **336**:348-352 (1988), describe a general strategy for targeting mutations to non-selectable genes. Weidle *et al.,* Gene, **6**6:193-203, (1988), describe amplification of tissue-type plasminogen activator with a DHFR gene and loss of amplification in the absence of selective pressure. Murnane and Yezzi, Somatic Cell and Molecular Genetics, **14**:273-286, (1988), describe transformation of a human cell line with an integrated selectable gene marker lacking a transcriptional promoter, with tandem duplication and amplification of the gene marker. Thomas and Capecchi, Cell, **51**:503-512, (1987), describe site-directed mutagenesis by gene targeting in mouse embryo-derived stem cells. Song *et al.,* Proc. Natl. Acad. Sci. USA, **84**:6820-6824, (1987), describe homologous recombination in human cells by a two staged integration. Liskay *et al.,* "Homologous Recombination Between Repeated Chromosomal Sequences in Mouse Cells," Cold Spring Harbor, Symp. Quant. Biol. **49**:13-189, (1984), describe integration of two different mutations of the same gene and homologous recombination between the mutant genes. Rubnitz and Subramani, Mol. and Cell. Biol. **4**:2253-2258, (1984), describe the minimum amount of homology required for homologous recombination in mammalian cells. Kim and Smithies, Nucl. Acids. Res. **16**:8887-8903, (1988), describe an assay for homologous recombination using the polymerase chain reaction.

Burke, *et al*., Science **236**:806-812 (1987) describe yeast artificial chromosomes (YACs). See also, Garza, *et al*., Science **246**:641-646 (1989) and Brownstein, *et al*. Science **244**:1348-1351 (1989).

See also, U.S. Application Serial No. 432,069, filed November 6, 1989 and Serial Nos. 466,088, filed January 12, 1990 and 610,515, filed November 11, 1990, which applications are incorporated herein by reference.

### SUMMARY OF THE INVENTION

Expression of mammalian proteins is achieved by homologous recombination, where a DNA sequence is integrated into the genome or large fragment thereof for enhancing the expression of the target gene. The modified sequence may then be transferred to a secondary host for expression. Where an amplifiable gene is integrated adjacent to the target gene, the target region may be amplified for enhanced expression.

Two different targets may be employed: homologous recombination in a host cell comprising the wild type target gene; or integration into a selected YAC(s) or YAC library and transfer of the target region to the expression host. When using a YAC or YAC genomic library containing mammalian DNA, particularly human, the gene of interest is manipulated by homologous recombination, which includes the introduction of an amplifiable gene in proximity to the target gene to allow for amplification, and may in addition include, depending upon the expression host, modification of the transcriptional system, and modification(s) of the coding region. Depending upon whether the gene can be expressed in the yeast host, usually the YAC will be transformed into a mammalian cell expression host for integration and expression of the target gene. Amplification can now be induced and cells selected for stable high levels of expression of the target protein.

### DESCRIPTION OF THE SPECIFIC EMBODIMENTS

Methods and compositions are provided for production of mammalian proteins of interest in culture, particularly where one wishes to manipulate the native transcriptional system and/or coding region. The method employs integrating DNA by homologous recombination into genomic DNA, either in the native primary host cell or in a yeast primary host cell, using YAC(s) or YAC genomic library as the source of the target gene. The former technique is described in Patent Application Serial No. 432,069, filed November 6, 1989.

For transformation of the primary host cell, the cells may be grown and transformed with the DNA targeting construct, using any of a variety of selection techniques for selecting cells having the proper integration. Usually, there will be only one or two integration steps. For the most part, the constructs and techniques employed will be the same for targeting a gene in a chromosome of a native cell or targeting a gene in a large genomic fragment in a YAC.

The YAC library is maintained and propagated in a yeast host and homologous recombination is then employed for integrating a DNA targeting construct, usually comprising an amplifiable gene for integration into a target region comprising the target gene, which target gene encodes the protein of interest, while also allowing for, in the same or separate step, manipulation of the transcriptional system and/or the coding region. The modified yeast cells may then be analyzed and sequences providing for the desired modifications identified. The amplifiable region may then, as appropriate, be transformed into the expression host and the amplifiable region amplified.

"Transform" includes transform, transfect, transduce, conjugate, fuse, electroporate or any other technique for introducing DNA into a viable cell.

After amplification, by employing the amplifiable gene, the transformed hosts are then screened for production of the target protein and stability and derivative cell lines are selected for desired levels of production, which cells may be expanded and used for production of the desired protein in culture.

The source of the DNA, as the primary cell or DNA in the YAC, may be any mammalian cell of interest, particularly mammalian cells which do not grow readily in culture, more particularly primate cells, especially human cells, where the human cells may be normal cells, including embryonic, or neoplastic cells, particularly normal cells. Various cell types may be employed as the primary cells, including fibroblasts, particularly diploid skin fibroblasts, keratinocytes, myoblasts, lymphocytes, glia, epithelial cells, neurons, endothelial cells, or other somatic cells, or germ cells. Of particular interest are skin fibroblasts, which can be readily propagated to provide for large numbers of normal cells, embryonic kidney cells, and the like. These cells may or may not be expressing the gene of interest. In those instances where the target gene is inducible or only expressed in certain differentiated cells, one may select cells in which the target gene is expressed, which may require immortalized cells capable of growth in culture.

A number of amplifiable genes exist, where by appropriate use of a selection agent, a gene integrated in the genome will be amplified with adjacent flanking DNA. Amplifiable genes include dihydrofolate reductase (DHFR, metallothionein-I and -II, preferably primate metallothionein genes, adenosine deaminase, ornithine decarboxylase, glutamine synthase, *etc.* The amplifiable gene will have transcriptional signals which are functional in the secondary or expression host and may be functional in the primary host, particularly where amplification is employed in the primary host or the amplifiable gene is used as a marker.

The target genes may be any gene of interest, there already having been a large number of proteins of interest identified and isolated with continual additions to the list. Proteins of interest include cytokines, such as interleukins 1-11; growth factors such as EGF, FGF, PDGF, and TGF somatotropins; growth hormones; or other hormones, such as FSH, LH, etc.; colony stimulating factors, such as G-, M-, and GM-CSF; erythropoietin; steel factor; receptor antagonists, such as IL-1rA; plasminogen activators, such as tissue and urine; enzymes, such as superoxide dismutase; interferon -α, -β, -γ or modifications thereof; T-cell receptors; surface membrane proteins; insulin; lipoproteins; α₁-antitrypsin; CD proteins, such as CD3, 4, 8, 19; clotting factors, *e.g.*, Factor VIIIc, IX, and von Willebrands factor; anticlotting factors, such as Protein C; atrial naturetic factor, tumor necrosis factor; transport proteins; homing receptors; addressins; regulatory proteins; *etc*.

The YACs are prepared in accordance with conventional ways. Genomic DNA is cleaved enzymatically, mechanically, or by other means to provide fragments which will usually have at least about 50 kbp, more usually at least about 100 kbp, conveniently at least about 200 kbp and usually not more than about 2,000 kbp, more usually not more than about 1,000 kbp. The genomic DNA is inserted into a YAC and then screened using appropriate probes for identifying the presence of the target gene. The presence of the YAC may be verified by a selective medium for the markers present on the YAC. Yeast cells containing a YAC or YAC library may be characterized by hybridization analyses or by the polymerase chain reaction (PCR) using primers. The identified YAC(s) may then be used for manipulation.

The YAC will normally be transferred from the original yeast host to a different yeast host which is convenient for manipulation. The new host will be a haploid or diploid strain having a plurality, usually at least 2, and may have 5 or more mutations in different genes which allow for selection by complementation. Total yeast DNA from the original yeast host may be transformed into yeast cells or spheroplasts yielding cells, which may serve as the hosts for the manipulations. The resulting transformants may be plated on selective media which selects against transformants lacking the complementation markers present on the YAC.

Alternatively, one may transfer the YAC by a genetic cross. The recipient host for manipulation will normally be either a haploid or diploid host having a genetic defect which will be complemented by the genotype of the original yeast strain. If diploid, the recipient host is sporulated and ascospores are mixed with the original yeast host on an appropriate medium to allow mating. If a haploid and of opposite mating type to the original host strain, cells may be mated directly. Hybrid diploids are selected on selective media, where only cross-hybrids grow due to complementation between the non-allelic auxotrophic markers. Hybrids may then be sporulated and either random spores selected, for example, using expression of the heterozygous recessive drug-resistance marker, can1, to select for haploid meiotic products, or tetrads dissected using a micromanipulator. The meiotic products may then be analyzed genetically for the presence of the YAC markers, as well as the genetic markers present in the recipient strain. The presence of the YAC may be confirmed by hybridization or PCR analyses.

The manipulation of the mammalian DNA sequence in the YAC may be achieved in accordance with known techniques for homologous recombination in yeast. Thus, the sequence to be integrated into the mammalian sequence will have a region of homology of at least about 50 bp, more usually at least about 200 bp, usually at least about 50 bp at one terminus of the sequence homologous with the recombination target region, more usually at least about 200 bp, and usually at least 5 bp at the other terminus. The greater homology will usually be at the 5'- terminus for the gene activation or 3' terminus for generating other modifications, such as protein fusions or modifications aimed at increasing mRNA stability. Preferably, there will be at least a total of 100 bp of homologous sequence, more preferably at least about 200 bp with at least about 50 bp at each terminus. The homologous sequence may be 1 kbp or more.

Various sequences having homology to the target region may be employed. In addition to using sequences unique to the target region one may also use sequences which are homologous to repetitive sequences found in the mammalian genome, such as the Alu, LINE, THE, etc. sequence, where on the targeting construct such sequence may be present in one or multiple copies, usually not more than about 10 copies. Alternatively, one may utilize sequences from a YAC arm, such as prokaryotic sequences associated with the YAC arm, genetic markers on the YAC which are absent from the yeast genome, or the like. Where homologous vector arm sequences are employed, one or more kb of homology may be used.

The use of these alternative sequences is particularly helpful when only a small amount of 5'-untranslated region or N-terminal amino acid sequence about the target gene information is known. The 3'-terminus will generally have at least about 20 bp of homology.

Alternatively, one may have one region of homology followed by other sequences to be inserted and a telomere as the construct, (referred to as a half-YAC) where a centromere may or may not be present.

The sequence to be integrated into the mammalian sequence may be introduced into the primary host by any convenient means, which includes calcium precipitated DNA, spheroplast fusion, transformation, electroporation, biolistics, lipofection, microinjection, or other convenient means. Where an amplifiable gene is being employed, the amplifiable gene may serve as the selection marker for selecting hosts into which the amplifiable gene has been introduced. Alternatively, one may include with the amplifiable gene another marker, such as a drug resistance marker, e.g. neomycin resistance (G418 in mammalian cells), hygromycin in resistance etc., or an auxotrophy marker (HIS3, TRP1, LEU2, URA3, ADE2, LYS2, etc.) for use in yeast cells.

Depending upon the nature of the modification and associated targeting coinstruct, various techniques may be employed for identifying targeted integration. Conveniently, the DNA may be digested with one or more restriction enzymes and the fragments probed with an appropriate DNA fragment which will identify the properly sized restriction fragment associated with integration.

Besides an amplifiable gene, other DNA sequences may be employed to enhance expression, either by themselves or in combination with the amplifiable gene. Thus, one may use different promoter sequences, enhancer sequences, or other sequence which will allow for enhanced levels of expression in the expression host. Thus, one may combine an enhancer from one source, a promoter region from another source, a 5'- noncoding region upstream from the initiation methionine from the same or different source as the other sequences, and the like. One may provide for an intron in the non-coding region with appropriate splice sites or for an alternative 3'- untranslated sequence or polyadenylation site. Depending upon the particular purpose of the modification, any of these sequences may be introduced, as desired.

Other modifications may also be included. With relatively small deletions, insertions, point mutations, and the like, where relatively small intends under 1 kbp, usually under 500 bp, the flanking region may include such modification, where desirably at least about 50 bp of homologous sequence is at the terminus. Thus, modifications may include introduction of an enhancer sequence, introduction or substitution of or removal of a signal leader, removal of a splice donor or accceptor or an intron modification thereof, changes in the coding sequence, such as deletions, insertions, or substitutions, where the substitution provides for a change in the amino acid, or combinations thereof. Where two non-contiguous mutations are to be introduced, depending upon the nature and site of the mutations, one may wish to provide for the mutations in two steps, where either of the two steps may be carried out first.

A wide variety of mutations may be of interest, not only as to modifications in the coding sequence, but also in the preparation of fusion proteins where, the target gene may be retained intact or a portion of the target gene may be substituted with the integrating sequence. A number of fusion proteins have found interest, where a constant region from a member of the immunoglobulin superfamily, particularly antibodies, more particularly A IgG isotype, may be fused to the target protein. Alternatively, one may wish to introduce an enzyme, metallothionein, homing receptor, glycoside recognition site, phospholipid recognition site, or the like. In this manner, one may modify the target protein to provide for desirable characteristics which are not naturally present with the target protein.

In carrying out one or multiple transformation steps, each step may be carried out in substantially the same way, except that one may choose to use various techniques other than selection at one or both steps. Where selection is intended, the sequence to be integrated will have with it a marker gene, which allows for selection. The marker gene may conveniently be downstream from the target gene and may include resistance to a cytotoxic agent, e.g. antibiotics, heavy metals, or the like, resistance or susceptibility to HAT, gancyclovir, etc., complementation to an auxotrophic host, particularly by using an auxotrophic yeast as the host for the subject manipulations, or the like. The marker gene may also be on a separate DNA molecule, particularly with primary mammalian cells. Alternatively, one may screen the various transformants, due to the high efficiency of recombination in yeast, by using hybridization analysis, PCR, sequencing, or the like.

Yeast are particularly susceptible to homologous recombination, with a high degree of efficiency. Thus, the subject methodology allows for modification of the target locus with a high success rate so that one or a plurality of homologous recombinations may be carried out to achieve any particular modeling of the transcriptional initiation system, the coding region, or other aspect of the sequence of interest, as well as introduction of other sequences in cis, *e.g.*, amplifiable genes.

For homologous recombination, constructs will be prepared where the amplifiable gene will be flanked, normally on both sides with DNA homologous with the DNA of the target region. Depending upon the nature of the integrating DNA and the purpose of the integration, the homologous DNA will generally be within 100 kb, usually 50 kb, preferably about 25 kb, of the transcribed region of the target gene, more preferably within 2 kb of the target gene. Where modeling of the gene is intended, homology will usually be present proximal to the site of the mutation. By gene is intended the coding region and those sequences required for transcription of a mature mRNA. The homologous DNA may include the 5'-upstream region outside of the transcriptional regulatory region or comprising any enhancer sequences, transcriptional initiation sequences, adjacent sequences, or the like. The homologous region may include a portion of the coding region, where the coding region may be comprised only of an open reading frame or combination of exons and introns. The homologous region may comprise all or a portion of an intron, where all or a portion of one or more exons may also be present. Alternatively, the homologous region may comprise the 3'-region, so as to comprise all or a portion of the transcriptional termination region, or the region 3' of this region. The homologous regions may extend over all or a portion of the target gene or be outside the target gene comprising all or a portion of the transcriptional regulatory regions and/or the structural gene.

In the case of the amplifiable gene, the homologous sequence will be joined to the amplifiable gene, proximally or distally. Usually a sequence other than the wild-type sequence normally associated with the target gene will be used to separate the homologous sequence from the amplifiable gene on at least one side of the amplifiable gene. Some portion of the sequence may be the 5' or 3' sequence associated with the amplifiable gene, as a result of the manipulations associated with the amplifiable gene.

The integrating constructs may be prepared in accordance with conventional ways, where sequences may be synthesized, isolated from natural sources, manipulated, cloned, ligated, subjected to *in vitro* mutagenesis, primer repair, or the like. At various stages, the joined sequences may be cloned, and analyzed by restriction analysis, sequencing, or the like. Usually during the preparation of a construct where various fragments are joined, the fragments, intermediate constructs and constructs will be carried on a cloning vector comprising a replication system functional in a prokaryotic host, *e.g.*, E. coli, and a marker for selection, e.g., biocide resistance, complementation to an auxotrophic host, etc. Other functional sequences may also be present, such as polylinkers, for ease of introduction and excision of the construct or portions thereof, or the like. A large number of cloning vectors are available such as pBR322, the pUC series, etc. These constructs may then be used for integration into the primary mammalian host or yeast containing YAC.

In the case of the primary mammalian host, a replicating vector may be used. Usually, such vector will have a viral replication system, such as SV40, bovine papilloma virus, adenovirus, or the like. The linear DNA sequence vector may also have a selectable marker for identifying transfected cells. Selectable markers include the neo gene, allowing for selection with G418, the herpes tk gene for selection with HAT medium, the gpt gene with mycophenolic acid, complementation of an auxotrophic host, etc.

The vector may or may not be capable of stable maintenance in the host. Where the vector is capable of stable maintenance, the cells will be screened for homologous integration of the vector into the genome of the host, where various techniques for curing the cells may be employed. Where the vector is not capable of stable maintenance, for example, where a temperature sensitive replication system is employed, one may change the temperature from the permissive temperature to the non-permissive temperature, so that the cells may be cured of the vector. In this case, only those cells having integration of the construct comprising the amplifiable gene and, when present, the selectable marker, will be able to survive selection.

Where a selectable marker is present, one may select for the presence of the targeting construct by means of the selectable marker. Where the selectable marker is not present, one may select for the presence of the construct by the amplifiable gene. For the neo gene or the herpes tk gene, one could employ a medium for growth of the transformants of about 0.1-1 mg/ml of G418 or may use HAT medium, respectively. Where DHFR is the amplifiable gene, the selective medium may include from about 0.01-0.5 µM of methotrexate or be deficient in glycine-hypoxanthinethymidine and have dialysed serum (GHT media).

In carrying out the homologous recombination, the DNA will be introduced into the expression host. Techniques which may be used include calcium phosphate/DNA coprecipitates, microinjection of DNA into the nucleus, electroporation, yeast protoplast fusion with intact cells, transfection, polycations, e.g., polybrene, polyornithine, etc., or the like. The DNA may be single or double stranded DNA, linear or circular. For various techniques for transforming mammalian cells, see Keown *et al*., Methods in Enzymology (1989), Keown *et al*., Methods and Enzymology (1990) Vol. 185, pp. 527-537 and Mansour *et al.,* Nature, **336**:348-352, (1988).

Upstream and/or downstream from the target region construct may be a gene which provides for identification of whether a double crossover has occurred. For this purpose, the herpes simplex virus thymidine kinase gene may be employed since the presence of the thymidine kinase gene may be detected by the use of nucleoside analogs, such as acyclovir or gancyclovir, for their cytotoxic effects on cells that contain or lack a functional HSV-tk gene. The absence of sensitivity to these nucleoside analogs indicates the absence of the thymidine kinase and, therefore, where homologous recombination has occurred, that a double crossover event has also occurred.

Once the target region has been modified and the presence of the appropriate modifications established using restriction analysis, sequencing, hybridization, PCR etc., the manipulated YAC may then be used directly or may be further manipulated to reduce its size, e.g., restriction digestion or targeted fragmentation with a repeated mammalian sequences.

It may be desirable to increase the number of copies of the YAC per yeast cell in order to increase the efficiency of the transfer into mammalian cells. One may use a YAC with its appropriate host strain that allows a multi-fold amplification of the YAC. See, for example, Smith *et al.,* PNAS (1990) **87:**8242-8246. The YAC may be manipulated, as appropriate, to provide for appropriate markers for introduction of the construct into the amplifiable YAC. The amplifiable YAC, when amplified, may also find use to improve the efficiency of gene targeting and homologous recombination.

Various secondary mammalian expression hosts are available and may be employed. These hosts include CHO cells, particularly DHFR deficient cells, monkey kidney cells, C127 mouse fibroblasts, 3T3 mouse cells, Vero cells, etc. In the case of amplification, desirably the hosts will have a negative background for the amplifiable gene or an amplifiable gene which is substantially less responsive to the amplifying agent.

In the presence of a marker, the transformed cells are grown in selective medium containing, for the DHFR gene about 0.01-0.5 µM methotrexate or GHT media with dialyzed serum and, where another marker is present, e.g., the neo gene, the medium may contain from about 0.1-1 mg/ml G418. The resistant colonies are isolated and may then be analyzed for the presence of the construct in juxtaposition to the target gene. This may be as a result of detection of expression of the target gene product, where there will normally be a negative background for the target gene product, use of PCR, Southern hybridization, or the like.

The cells containing the amplifying construct are then expanded and subjected to selection and amplification with media containing progressively higher concentrations of the amplifying reagent, for example, 0.1-200 µM of methotrexate for the DHFR gene, and may be analyzed at each selection step for production of the target product. Expansion will include at least duplication and may result in at least 5 copies, preferably 10 copies or more in a tandem relationship. Thus protein production will be increased at least 1.5 fold from expression from a single copy, usually at least 3 fold, preferably at least 5 fold.

The various clones may then be screened for optimum stable production of the target product and these clones may then be expanded and used commercially for production in culture. In this manner, high yields of a product may be obtained, without the necessity of isolating the message and doing the various manipulations associated with genetic engineering or isolating the genomic gene, where very large genes can be a major research and development effort.

The following examples are offered by way of illustration and not by way of limitation.

### EXPERIMENTAL

### Activation of FSH-β gene expression

Construction of a FSH-β gene targeting vector: To activate the expression of the FSH-β gene, a YAC targeting vector (pYFT1) is constructed containing the following elements (5' to 3'): a 5' targeting region consisting of nucleotides -452 to -36 of the FSH-β gene (Jameson, *et al*. Mol. Endocrinology. (1988) **2**:806-815), an FSH-α cDNA expression cassette, a dihydrofolate reductase (DHFR) expression cassette, the yeast selectable marker LEU2, the human cytomegalovirus immediate early (CMV IE) region enhancer/promoter/splice donor sequences, and a 3' targeting region consisting of nucleotides +100 to +850 of the FSH-β, gene. This plasmid is derived from the plasmids pTD-F and pMF-F. pTD-F is constructed by three successive fragment insertions into pTD, which is created by inserting the synthetic polylinker 5'-Bsu36I-KpnI-MluI-XhoI-Bsu36I-3' into pSKII (Stratagene) between the KpnI and SacI sites, with the loss of those sites. First, the 1.95 kb PvuII/BamHI fragment of SV2DHFR (Subramani, *et al*. Mol. Cell. Biol. (1981) **1**:854-864) encoding the SV40 early promoter, the DHFR gene, the SV40 t antigen intron and the SV40 early polyadenylation site is joined to MluI linkers and cloned into the MluI site. Second, the 2.0 kb BssHII fragment of pIKFSH-α (described below) encoding an FSH-α cDNA expression cassette is joined to KpnI linkers and cloned into the KpnI site. Finally, the 2.4 kb BglII/SalI fragment of YEpl3 (ATCC 37115) encoding the yeast selectable marker LEU2 is cloned between a BamHI site at the 3' end of SV2 DHFR and the XhoI site. The order of the three elements within the Bsu36I cassette is 5'-pIKFSHα-SVDHFR-LEU2-3', each having the same transcriptional orientation.

pIKFSH-α was generated by inserting an FSH-α cDNA between the BglII and ApaI sites of pIK (see below). The cDNA was cloned by reverse transcription of 0.2 µg of total RNA from the cell line CHA/GO K-1 (ATCC HTB 168) primed by pdN₆ (Pharmacia/LKB), followed by polymerase chain reaction (PCR) with primers 1 and 2. A cDNA clone was obtained which encodes the complete FSH-α coding sequence (Fiddes and Goodman 1981, J. Mol. Appl. Gen. **1**, 3-18). pIK is a mammalian expression vector constructed by four successive cassette insertions into pMF2, which was created by inserting the synthetic polylinker 5'-HindIII-SphI-EcoRI-AatII-BglI-XhoI-3' into KpnI and SacI sites of pSKII, with loss of the Kpn I and Sac I sites. First, a BamHI-XbaI fragment containing the SV40 T antigen polyadenylation site (nucleotides 2770 to 2533 of SV40, Reddy *et al*. 1978, Science **200**, 494-502) and an NheI-SalI fragment containing the SV40 origin of replication (nucleotides 5725 to 5578 of SV40) were inserted by three-part ligation between the BglII and XhoI sites, with the loss of the BglII, BamHI, XbaI, NheI, SalI and XhoI sites. These BamHI-XbaI and NheI-SalI fragments were synthesized by PCR with pSV2neo (Southern and Berg 1982, J. Mol. Appl. Gen. **1**, 327-341) as the template using oligonucleotide primer pairs 3 and 4, and 5 and 6, respectively, which incorporated BamHI, XbaI, NheI and SalI sites at their respective ends. Second, an SphI-EcoRI fragment containing the splice acceptor of the human α1 globin gene second exon (nucleotides +143 to +251) was inserted between the SphI and EcoRI sites. This SphI-EcoRI fragment was synthesized by PCR with ppSVaHP (Treisman *et al*. 1983, PNAS **80**, 7428-7432) as the template using oligonucleotide primers 7 and 8, which incorporated SphI and EcoRI sites at their respective ends. Third, the synthetic polylinker 5'-EcoRI-BglII-ApaI-AatII-3' was inserted between the EcoRI and the AatII sites. Fourth, a HindIII-XbaI fragment containing the CMV IE enhancer/promoter (nucleotides -674 to -1, Boshart *et al*. 1985, Cell **41**, 521-530) and a XbaI-SphI fragment containing the CMV IE first exon/splice donor (nucleotides +7 to +170) are inserted by three-part ligation between the HindIII and SphI sites. The HindIII-XbaI fragment is prepared by PCR with pUCH.CMV (M. Calos, Stanford Univ.) as the template using oligonucleotide primers 9 and 10, which incorporated HindIII and XbaI sites at their respective ends. The XbaI-SphI fragment is chemically synthesized.

pMF-F is constructed by three successive fragment insertions into pMF, which is created by inserting the synthetic polylinker 5'-NheI-Bsu36I-HindIII-SphI-NotI-3' into pSKII between the KpnI and SacI sites, with the loss of the those sites. First, a NheI-Bsu36I fragment containing nucleotides -452 to -36 of the FSH-β gene (5' targeting region) is synthesized by PCR with DNA from the human diploid fibroblast line WI38 (ATCC CCL 75) as the substrate and the oligonucleotide primers 11 and 12 and cloned between the NheI and Bsu36I sites. Second, an SphI-NotI fragment containing nucleotides +100 to +850 of the FSH-β gene (3' targeting region) is synthesized as described above with primers 13 and 14 and cloned between SphI and NotI sites. Finally, an 840 bp HindIII-XbaI cassette isolated from pIK, containing the CMV IE enhancer, promoter, first exon and splice donor is cloned between the HindIII and SphI sites.

pYFT1 is constructed by inserting the 6.2 kb Bsu36I fragment of pTD-F into the unique Bsu36I site of pMF-F, with the transcriptional orientation of the elements within the Bsu36I fragment identical to the CMV IE enhancer/promoter in pMF-F.

A second YAC targeting vector (pYFT2) for FSH-β gene activation is constructed by inserting the 6.2 Kb Bsu361 fragment of pTD-F into the unique Bsu361 site of pMF-F2. pMF-F2 is constructed by two successive fragment insertions into pMF. First, the NheI-Bsu361 fragment containing nucleotides -452 to -36 of the FSH-β gene is cloned between the NheI and Bsu361 sites. Second, a 680 bp HindIII-XbaI cassette isolated from pIK containing the CMV IE enhancer and promoter, and an XbaI-NotI fragment containing nucleotides +7 to +850 of the FSH-β gene (3' targeting region) are inserted by three-part ligation between the HindIII and NotI sites. The Xba-NotI fragment is synthesized by PCR using oligonucleotide primers 13a and 14, which incorporate XbaI and NotI sites as their respective ends.

Oligonucleotide primers:

Primer 1:     5'-GAATTCAGATCTGCAGTTACTGAGAACTCATAAG-3'

Primer 2:     5'-GAATTCGGGCCCTGCAGTGGAACAAGCTTAATG-3'

Primer 3:     5'-GGTCGACCTGGATCCGCCATACCACATTTGTAG-3'

Primer 4:     5'-GCCGCGGCTCTAGAGCCAGACATGATAAGATAC-3'

Primer 5:    5'-AAGCTTGTGCTAGCTATCCCGCCCCTAACTCCG-3'

Primer 6:    5'-CGAATTCGGTCGACCGCAAAAGCCTAGGCCTCC-3'

Primer 7:     5'-GTCTATAGCATGCTCCCCTGCTCCGACCCG-3'

Primer 8:     5'-GGTACCGAATTCTCCTGCGGGAGAAGCAG-3'

Primer 9:     5'-CGCCAAGCTTGGCCATTGCATACGGT-3'

Primer 10:    5'-GAGGTCTAGACGGTTCACTAAACGAGCTCT-3'

Primer 11:    5'-GAATTCGCTAGCGACAGGAGCCAGATCATGAAATG-3'

Primer 12:    5'-CCATGGCCTGAGGTCATGTGCAACTAACACCTTGT-3'

Primer 13:    5′-GAATTCGCATGCGGCATGGAGGACAAAACTAGAG-3′

Primer 13a:  5'-GAATTCTCTAGAACAGCTCTTGCCAGGCAAGGCA-3'

Primer 14:    5'-GGATCCGCGGCCGCGCCCACTAGAAACTGAGAAACC-3'

Primer 15:    5'-GAATTCAGATCTGGTACCATGTTTTGCTGGAAGC-3'

Primer 16:    5'-GGATCCGAGCTCTTGAGGAGTTTAAGAAGAGAGTT-3'

YAC screening: Two yeast artificial chromosome (YAC) libraries are screened to identify YACs containing the human FSH-β locus. Each YAC library is generated in the haploid Saccharomyces cerevisiae host strain AB1380 [MAT a ade2-1 (ochre) lys2-1 (ochre) trpl ura3 his5 canl-100 (ochre)] using pYAC4 (Burke *et al*. Science (1987) **236**:806-812). The YAC contains two yeast selectable markers, TRP1 and URA3. Thus, the presence of a YAC in strain AB1380 is verified genetically by scoring for the presence of the wildtype TRP1 and URA alleles. The CEPH YAC library (Albertsen *et al.* 1990, **PNAS 87**:4256-4260), which consists of -50,000 colonies with an average YAC insert size of 430 kb (-7 haploid genome equivalents), is screened by PCR. A total of 113 pools of DNA, each prepared from ~386 yeast colonies, are screened with FSH-β primers 11 and 12 (see above) which generate a 416 bp fragment. The Washington University YAC library (Brownstein *et al*., Science (1989) **244**:1348-1351), which consists of -60,000 colonies with an average YAC insert size of 250 kb (-5 haploid genome equivalents), is screened by hybridization to filters prepared from pulsed-field gels. The YAC colonies are arrayed in pools of -386 colonies, chromosomal DNA is prepared from each pool and the DNA is separated on pulsed-field gels. The 416 bp FSH-β PCR product prepared with primers 11 and 12 is used as a hybridization probe (designated FSH 5' probe). Yeast colony hybridization to filters prepared from individual YAC colonies (Traver, Klapholz, Hyman and Davis 1989; PNAS **86**:5898-5902) is used to screen for FSH-β-containing YACs from each of the positive pools. Several positive YAC colonies are identified and analyzed by Southern blot analysis for the presence of characteristic hybridizing EcoRI, BglII, BamHI and NsiI fragments using the FSH 5' probe. One designated YAC-FSH-β1, having the expected pattern, is chosen for subsequent experimentation.

Transfer of YAC-FSH-β1 to a new host: All yeast genetic manipulations employ standard methodology essentially as described in Sherman, Fink and Hicks (1986, Laboratory Course Manual for Methods in Yeast Genetics). To efficiently carry out homologous targeting of YAC-FSH-β1, the YAC is transferred to a new haploid yeast host strain, YPH252 (MATα ade2-101 (ochre) lys2-801 (amber) ura3-52 trp1Δ1 his3Δ200 1eu2Δ1) (Sikorski and Hieter 1989, Genetics **122**:19-27) or YPH274 (MATa/MATα ade2-101/ade2-101 lys2-801/lys2-801 ura3-52/ura3-52 trp1Δ1/trp1Δ1 his3Δ200/his3Δ200 leu2Δ1/leu2Δ1) (Sikorski and Hieter, abid). The YPH252 host contains nonreverting alleles of ura3, trpl, leu2 and his3; with the latter three being deletion alleles. The latter host contains nonreverting alleles of ura3, trp1, leu2 and his3; with the latter three being deletion alleles. Total yeast chromosomal DNA is prepared in agarose plugs using standard methodology (McCormick *et al*., Technique (1990) 2:65-71). The agarose plugs are equilibrated in 50 mM NaCl; 10 mM Tris-HCl pH 7.5; 0.75 mM spermidine trihydrochloride; 0.3 mM spermine tetrahydrochloride, melted at 65°C and used to transform yeast spheroplasts of the strain YPH252 or YPH274 (Burgers and Percival, Anal. Biochem. (1987) **163**:321-397; McCormick *et al*., J. Methods in Cell and Mol. Biol. (1990) **2**:65-71). Transformants are plated on media lacking uracil to select for the presence of the wildtype URA3 marker contained on the YAC. The presence of the YAC is confirmed by the additional presence of the wildtype TRP1 allele. Alternatively, the YAC-FSHβ1 in AB1380 is mated to yeast strain YPH252. Diploid cross hybrids, selected on media lacking histidine, are sporulated, patched into a selective medium containing canavanine enriching haploid meiotic products expressing the recessive drug resistance marker can1-100. Canavanine resistant colonies are genetically screened for the presence of the leu2Δl allele and the YAC.

Gene targeting of the FSH-β locus: The targeting vectors pYFT1 and pYFT2 are digested with NheI and NotI to liberate -7.3 kb fragments. These fragments are used to transform yeast spheroplasts of the strain YPH252/YAC-FSH-β1 or YPH274/YAC-FSHβ1 using the lithium acetate method (Schiestl and Gretz, 1989, Current Genetics **16**:339-346). Total yeast genomic DNA from Leu⁺ transformants is subjected to Southern blot analysis and compared with DNA from untransformed cells to detect targeting of the FSH-β locus. DNA is digested with NheI, SacI, EcoRI, BglII, BamHI or NsiI and probed with a ∼700 bp fragment from the second intron of the FSH-β gene lying outside of the targeting vector (designated FSH 3' probe). This probe is synthesized by PCR with DNA from WI38 cells as the template using oligonucleotide primers 15 and 16. The resulting -1.4 kb fragment is digested with PstI and SacI to generate the ∼700 bp fragment. Correctly targeted colonies display hybridizing fragments consistent with the insertion of the -7.0 kb corresponding to pIKFSHα, SVDHFR, LEU2 AND CMV enhancer and promoter sequence.

### Transfer of the targeted FSH-β locus into CHO cells:

Total yeast DNA is used to transfect CHO DHFR-DUKX B 1 (Urlaub and Chasin, 1990, PNAS 7:4216-4220) cells essentially as described by Eliceiri *et al.* for YAC transfer into Mouse L/tk- cells (Eliceiri *et al.* 1991, PNAS **88**:2179-2183), but optimized for the transfection of CHO cells. Genomic DNA is extracted from the targeted cells by spheroplasting 5x10¹⁰ yeast cells as described by Phillipsen *et al.,* 1991, Methods in Enzymology **194**:169-174, followed by lysis in 20 ml 0.5% SDS, 10 mM Tris pH 7.5, 2 mM EDTA and 0.5 mg/ml Proteinase K and incubation at 50°C for 2-3 hr. After phenol and chloroform extraction, the lysate is adjusted to 100 mM NaCl, genomic DNA precipitated with isopropanol, resuspended, adjusted to 20 µg/ml RNAse A and digested for 30 min. at 37°C. The DNA is phenol and chloroform extracted, adjusted to 100 mM NaCl, precipitated with an equal volume of isopropanol, resuspended in 5 ml 10 mM Tris pH 7.5, 1 mM EDTA and then dialysed (13 changes, 4 L 10 mM Tris pH 7.5, 1 mM EDTA) for 48 h, followed by concentration by isopropanol precipitation as described above and resuspended at a final concentration of 0.5 µg/µl. 2x10⁵ CHO DHFR-cells are plated 12 hr prior to transfection on 10 cm dishes in DME/F12 media supplemented with 10% fetal bovine serum, glycine, hypoxanthine and thymidine (nonselective media). For transfection, 150µl of 2xHeBS (280 mM NaCl, 50 mM Hepes pH 7.1, 1.5 Na₂HPO₄) is added to 15 µg of yeast genomic DNA in 150 µl of 0.27M CaCl₂. The precipitate is allowed to form at room temperature for 25 minutes, added to cells in the absence of media and incubated for 20 minutes at room temperature, then topped off with 3 ml of media and incubated at 37°C.

Alternatively, 30-50 µg YAC containing yeast genomic DNA is diluted to 0.5 ml with sterile water, 0.5 ml of 0.5 M CaCl₂ added, followed by immediate addition of 1.0 ml HNP (49 mM Hepes pH 7.1, 274 mM NaCl, 1.4 mM Na₂ HPO₄). 2 ml of mix is added per 10 cm dish.

Four hours later, the cells are washed twice with serum-free media, incubated with 15% glycerol in HeBS for 4 minutes at 37°C, washed again in serum-free media followed by growth in nonselective media for 48 hours. The transfected cells are then split 1:20 into selective media in (DME/F12 supplemented with 10% dialyzed fetal bovine serum) and fed every 3 days. Transfected DNA in CHO DHFR+ transformants is amplified by selection in increasing concentrations of methotrexate. YAC containing CHO DHFR+ clones are plated in selective media at 5x10⁵ cells per 10 cm plate and selected at 0.01 µm methotrexate (Kaufman and Sharp, 1982. J. Mol. Biol. **159**:601-621). surviving colonies are pooled, then tested for increased DNA copy number by Southern blot analysis and increased protein production by immunoprecipitation analysis. The amplification protocol is repeated with increasing concentrations of methotrexate from 0.01 µm to 50 µM, with a three to five-fold increased methotrexate concentration at each step.

Analysis of CHO transfectants: Transfected colonies are expanded and characterized for the production of FSH-α and -β mRNA and biologically active heterodimeric FSH. Total RNA is prepared and the level of correctly initiated FSH-α and FSH-β mRNA is assayed by primer extension (Finer *et al.,* 1987) using the following oligonucleotides:

FSH-α: 5'-AGCTGCATATTTTCTGTAGTAATCC

FSH-β: 5′-CCTGGTGTAGCAGTAGCCAGCACAC

RNA expression is indicated by the detection of primer extension products consistent with the addition of the CMV first exon and polylinker sequence of pIK for FSH-α, and the exchange of the FSH-β first exon by the first exon of the CMV IE region.

Heterodimeric FSH is detected by pulse labeling of transfected clones with ³⁵S-methionine followed by immunoprecipitation of protein collected from the conditioned media and from cell lysates (Keene *et al.* 1989, JBC 264, 4769-4775). Analysis on native and denaturing polyacrylamide gels reveals a protein product similar to highly purified human FSH. Biological activity of the expressed product is confirmed using the *in vitro* granulosa cell aromatase bioassay (Jia *et al*., J. Clin. Endocrinol. Metab. (1986) **62**:1243-1249; Jia *et al.,* Endocrinology (1986) **119**:1570-1577).

### Activation of G-CSF Analog Expression

Construction of G-CSF targeting vectors: To activate the expression of the G-CSF gene, a YAC targeting vector (pYGT1) is constructed containing the following elements (5' to 3'): a 5' targeting region consisting of nucleotides -361 to -69 of the G-CSF gene (Nagata *et al.,* EMBO J. (1986) **5**:575-581), a DHFR expression cassette, the yeast selectable marker LEU2, the human CMV IE enhancer/promoter/splice donor, a human α-1 globin gene splice acceptor, and a 3' targeting region consisting of nucleotides -60 to +167 of the G-CSF gene. This plasmid is derived from plasmids pTD-G and pMF-G. Plasmid pTD-G is constructed by two successive fragment insertions into pTD. First, the 1.95 kb PvuII-BamHI fragment of SV2DHFR encoding the SV40 early promoter, the DHFR gene, the SV40 t antigen intron and the SV40 early polyadenylation site is joined to MluI linkers and cloned into the MluI site. Second, the 2.2 kb SalI-XhoI fragment of YEp13 encoding the yeast selectable marker LEU2 is cloned into the XhoI site. The order of these elements within the Bsu36I cassette was 5'-SV2DHFR-LEU2-3', both having the same transcriptional orientation.

pMF-G is constructed by the insertion into pMF of three fragments in two steps. First, an NheI-Bsu36I fragment containing nucleotides -361 to 69 of the human G-CSF gene (5' targeting region) is generated by PCR using oligonucleotide primers 17 and 18, and cloned between the NheI and Bsu36I sites. Second, an EcoRI-NotI fragment containing nucleotides -60 to +167 of the G-CSF gene is generated by PCR using oligonucleotide primers 19 and 20. This EcoRI-NotI fragment and a HindIII-EcoRI fragment from pIK containing the CMV IE enhancer/promoter/splice donor and human α-1 globin splice acceptor, are inserted by three-part ligation between the HindIII and NotI sites.

pYGT1 is constructed by inserting the 4.2 kb Bsu36I fragment of pTD-G into the unique·Bsu36I site of pMF-G, with the transcriptional orientation of the elements within the Bsu36I fragment identical to the CMV enhancer/promoter in pMF-G.

To create sequences capable of directing the modification of the G-CSF polypeptide, a YAC targeting vector (pYGT2) is constructed containing the following elements (5' to 3'): a 5' targeting region consisting of nucleotides +1180 to +1480 of the G-CSF gene, an IgG2 heavy chain cDNA encoding the hinge, CH2 and CH3 domains (amino acids 216-478, Kabat *et al.* 1983, Sequences of Proteins of Immunological Interest), an SV40 early polyadenylation site, the yeast selectable marker HIS3, and a 3' targeting region consisting of nucleotides +1496 to +2599 of the G-CSF gene. The 5' targeting sequences and IgG2 cDNA sequences are configured such that upon successful targeting a sequence encoding a hybrid G-CSF-IgG2 protein would be created in which Gln-176 of G-CSF is fused to Glu-216 of the IgG2 hinge region. pYGT2 is constructed by four successive fragment insertions into pDS, which is created by inserting the synthetic polylinker 5'-XbaI-MluI-BamHI-SphI-SalI-3' into pSKII between the KpnI and SacI sites, with the loss of those sites. First, an MluI-BamHI fragment containing the SV40 early polyadenylation site (nucleotides 2270 to 2533) is generated by PCR with pSV2DHFR as template using oligonucleotide primers 21 and 22 and inserted between the MluI and BamHI sites. Second, the 1.7 kb BamHI fragment of pNN414 encoding the yeast selectable marker HIS3 (Traver *et al*., *supra*) is cloned into the BamHI site. Third, an XbaI to blunt-ended fragment containing nucleotides +1180 to +1480 of the G-CSF gene, and a blunt-ended to MluI fragment encoding amino acids 216-478 of the IgG2 heavy chain are inserted in a three-part ligation between the XbaI and MluI sites. The G-CSF gene fragment is generated by PCR using oligonucleotide primers 23 and 24, and the IgG2 fragment is generated by PCR with a cDNA clone obtained from a human spleen cDNA library (Clontech) as template using oligonucleotide primers 25 and 26. Finally, a 1.1 kb SphI-SalI fragment containing +1496 to +2599 of the G-CSF gene, generated by PCR using oligonucleotide primers 27 and 28 is inserted between the SphI and SalI sites.

Primer 17: 5'-GAATTCGCTAGCCTGCCGCTTCCAGGCGTC-3'

Primer 18:  5'-GAATTCCCTAAGGCATAACCCCATGGAGGCC-3'

Primer 19: 5'-GATGATGAATTCGCCCCCTAGAGCTGGGCC-3'

Primer 20: 5'-ATGATGGCGGCCGCCCCTCTCGGGGACACTGG-3'

Primer 21:  5′-AGAGAGACGCGTGCCATACCACATTTGTAG-3′

Primer 22:  5'-GCAGCAGGATCCAGACATGATAAGATAC-3'

Primer 23: 5'-GAATTCTCTAGAAAGGTCGTGCTGGCATTC-3'

Primer 24:  5′-CTGGGCAAGGTGGCGTAG-3′

Primer 25:  5'-GAGCGCAAATGTTGTGTC-3'

Primer 26: 5'-GAATTCACGCGTCACGCGACCCCGAGAGCC-3'

Primer 27:  5'-AGAGAGGCATGCTCCCCATCCCATGTATTT-3'

Primer 28:  5'-GAATTCGTCGACCGAGTGCAGATTCCATGT-3'

Gene targeting of the G-CSF locus: Identification of a YAC colony containing the human G-CSF locus (YAC-G-CSF-1) using the 1.1 kb SphI-SalI fragment as probe and transfer of the YAC to yeast strain YPH252 are carried out as described above for the FSH-β locus. To activate the expression of a GCS-F-IgG2 hybrid polypeptide, two successive gene targeting events are carried out using the targeting vectors pYGT1 and pYGT2. First, the targeting vector pYGT1 is digested with NheI and NotI to liberate a 4.7 kb fragment. This fragment is used to transform yeast spheroplasts of the strain YPH252/YAC-G-CSF-1. Total yeast genomic DNA from Leu+ transformants is subjected to Southern blot analysis and compared with DNA from untransformed cells to detect targeting of the G-CSF locus. DNA is digested with restriction enzymes and probed with a -1.1 kb fragment from the 3' untranslated region of the G-CSF gene lying outside the targeted region. This probe is generated by PCR using oligonucleotide primers 27 and 28. Correctly targeted colonies display hybridizing fragments consistent with the insertion of the -4.7 kb corresponding to SVDHFR, Leu2 and CMV IE sequences. Next, the targeting vector pYGT2 is digested with XbaI and SalI to liberate a -4.1 kb fragment. This fragment is used to transform yeast spheroplasts of the strain created by the above targeting event. Total yeast genomic DNA from His+ transformants is subjected to Southern blot analysis and compared with DNA from untransformed cells to detect targeting of the G-CSF locus. DNA is digested with restriction enzymes and probed with a 300 bp fragment from the 5' untranslated region of the G-CSF gene lying outside the newly targeted region. This probe is generated by PCR using oligonucleotide primers 17 and 18. Correctly targeted colonies display hybridizing fragments consistent with the insertion of the ∼4.1 kb corresponding to IgG2, SV40 and HIS3 sequences. Total DNA is prepared from the doubly targeted yeast strain and used to transfect CHO DHFR⁻ cells as described above for the FSH-β gene. Following gene amplification of the transfected G-CSF-IgG2 sequences, CHO colonies are analyzed for expression of the G-CSF analog.

Analysis of YAC transfected CHO clones: Secreted G-CSF-IgG2 is characterized by labeling transfected clones with ³⁵S-methionine followed by immunoprecipitation of culture supernatants and cell lysates as described (Capon *et al.,* Nature (1989) **337**:525-531). Washed immunoprecipitates are eluted and electrophoresed on polyacrylamide gels under reducing conditions and visualized by autoradiography to reveal the hybrid polypeptide.

The presence of the G-CSF moiety is confirmed by Western blot analysis. Unlabelled supernatants are similarly immunoprecipitated and electrophoresed and the proteins transferred to nitrocellulose filters (Burnette, Anal. Biochem (1981) **112**:195) The filters are treated with a rabbit polyclonal anti-human-G-CSF antiserum (Genzyme) and the bands visualized by treating with Horseradish peroxidase-conjugated goat antirabbit-IgG antibody (Boehringer Mannheim) followed by staining with 3,3'-diaminobenzidine and H₂O₂.

The following example describes the use of a mammalian primary host cell for the target DNA.

### Activation of the Factor IX Gene:

Construction of a Factor IX Targeting Vector: Two YAC targeting plasmids, pRSN303.A/F9HR/340 and pRSN303.A/F9HR/34, were constructed to activate the expression of the Factor IX gene and contained the following elements 5' to 3': a 5' targeting region consisting of nucleotides -328 to +17 (pRSN303.A/F9HR/340) or -18 to +17 (pRSN303.A/F9HR/34) of the Human Factor IX gene (Yoshitake *et al.,* 1985, Biochemistry **24**:3736-3750), the yeast selectable marker His3 (Campbell, *et al.,* infra), the mammalian selectable marker MC1 neo (Thomas and Capecchi, 1987, Cell **51**:503-512), the plasmid backbone of pRS303 (Campbell *et al*., 1991, Proc. Natl. Acad. Sci., USA **88**:5744-5748), a mammalian selectable and amplifiable dihydrofolate reductase (DHFR) expression cassette from SV2 DHFR (Subramani *et al*. 1981, Mol. Cell. Biol. 1:854-864), the immediate early enhancer/promoter/first exon and splice donor of HCMV (Boshart *et al*., 1985, Cell **41**:521-530), the splice acceptor from intron 1 of the Human αl globin gene (Treisman *et al.,* 1983, PNAS **80**:7428-7432), and a 3' targeting region consisting of nucleotides +17 to +3996 of human Factor IX gene.

pRSN303.A/F9HR/340 was derived from plasmids pRSN303.A/340 and pSK.9/F9HR, and pRSN303.A/F9HR/34 was derived from plasmids pRSN303.A/34 and pSK.9/F9HR. pRSN303.A/340 was constructed by insertion of the synthetic 71-mer defined by oligonucleotides 1 and 2 between the Sac I and Apa I sites of pRSN303 (Campbell *et al.,* 1991, Proc. Natl. Acad. Sci. USA **88**:5744-5748), resulting in the loss of the Sac I site, to generate pRSN303.A. This oligonucleotide contained the restriction sites 5'-ApaI-ClaI-EcoRI-BclI-Espl-NotI-BstEII-NheI-3'. pRSN303 contains the yeast selectable marker His3 and the mammalian selectable marker MCIneo cloned in the plasmid pRS (Campbell, *et al*.). The 5' homology encoding nucleotides -322 to +17 of Human Factor IX were synthesized by PCR using pFIX-18 (Yoshitake *et al*., 1985, Biochemistry **24**:3736-3750) as a template and primers a and b. The product was cloned between the BstEII and Nhe I sites of pRSN303.A to generate pRSN303.A/340. pRSN303.A/34 was constructed by ligation of a synthetic 35-mer, defined by oligonucleotides by 7 and 8, between the Bst EII and Nhe I sites of pRSN303.A.

pSK.9/F9HR was constructed using two intermediates, pSK.9 and pCG.1. pSK.9 was constructed by insertion of the 71-mer described above into the Sac I and Apa I sites of Bluescript pSK- (Stratagene, pCG.1 was constructed by insertion of the synthetic 72-mer defined by oligonucleotides 3 and 4, batween the Sac I and Kpn I sites of pSK- (Stratagene), with the loss of tne Sac I and Kpn I sites. This oligonucleotide encoded the restriction sites 5'-NotI-SacII-ClaI-MluI-XhoI-HindIII-XbaI-BstEII-SacII-NotI-3'. A HindiII-XbaI fragment containing the CMV IE enhancer/promoter (nucleotides -674 to -1, Boshart *et al*. 1985, Cell **41**:521-530) was prepared by PCR with pUCH.CMV (M. Calos, Stanford Univ.) as the template using oligonucleotide primers c and d, which incorporated HindIII and XbaI sites at their respective ends. This fragment was cloned into pCG.1 to generate pCG.CMV. Next, a 1.94 kb Mlu I linkered SV2DHFR cassette was synthesized by PCR using the SV2 DHFR DNA as a template (Subramani *et al.,* 1981, Mol. Cell. Biol. **1**:854-864) and primers e and f. This fragment was cloned into the MluI site of pCG.CMV to generate pCG.CMV/DHFR. The 2.6 kb Cla I-Sac I fragment of pCG.CMV/DHFR, the 0.3 kb Sac I/Eco RI fragment of pIK were then ligated into the Cla I and Eco RI sites of pSK.9 to generate pSK.9/DHFR/CMV. Finally, a 4.0 kb Nhe I-Bgl II fragment of the human Factor IX gene was isolated from pFIX-18 (Yoshitake *et al.,* 1985, Biochemistry **24**:3736-3750) and ligated together with an Eco RI-Xba I adaptor, defined by oligonucleotides 5 and 6, into the Bcl I and Eco RI sites of pSK.9/DHFR/CMV to generate pSK.9/F9HR.

The final targeting plasmids were constructed by ligation of a 6.94 kb Cla I/Not I cassette from pSK.9/F9HR into Cla I/Not I cut pRSN303.A/340 or pRSN303.A/34 to yield pRSN303.A/F9HR/340 and pRSN303.A/F9HR/34, plasmids containing 340 bp or 34 bp, respectively, of Factor IX homology on the 5' end and 4 kb of homology on the 3' end of the vector, separated by the activation/amplification cassette.

### Oligonucleotides:

1. 5′-GTC GAC TGC TAG CAA TGG TGA CCT GCG GCC GCA GCT
GAG CGT GAT CAC ATG AAT TCC ATA TCG ATT GGG
CC-3′

2.    5′-CAA TCG ATA TGG AAT TCA TGT GAT CAC GCT CAG CTG
          CGG CCG CAG GTC ACC ATT GCT AGC AGT CGA CAG
          CT-3′

3.   5′-TGC GGC CGC GGT TAT CGA TGA CGC GTC CTC GAG CAA
        GCT TCT CTA GAC GGT CAC CTT CCG CGG CCG CTG
        TAC-3′

4. 5′-AGC GGC CGC GGA AGG TGA CCG TCT AGA GAA GCT TGC
TCG AGG ACG CGT CAT CGA TAA CCG CGG CCG CAA
GCT-3′

5. 5′-CTA GAG ATT GTG AAA G-3′

6.   5′-AAT TCT TTC ACA ATC T-3′

7.    5'-GTC ACG TAC AAC TAA TCG ACC TTA CCA CTT TCA CAA
      TCT G-3'

8.   5′-CTA GCA GAT TGT GAA AGT GGT AAG GTC GAT TAG TTG
TAC-3′

### PCR Primers:

a.   5′-CAG GT ACC GTA CAG CCA TTT TGG TAA ACA TCA T-3′

b.   5'-TTT GCT AGC AGA TTG TGA AAG TGG-3'

c. 5′-CGC CAA GCT TGG CCA TTG CAT ACG TT-3′

d.   5'-GAG GTC TAG ACG GTT CAC TAA ACG AGC TCT-3'

e.   5'-GGA CGC GTG GAT CCA GAC ATG ATA AGA TA-3'

f.    5'-GGA CGC GTC AGC TGT GGA ATG TGT GTC AG-3'

g.    5′-ACC ACT CAT ACA TTG CTG ATG G-3′

h.   5′-GGC CAG TGA ATT GTA ATA CG-3′

Targeting the Factor IX locus: The Factor IX YAC was targeted by lithium acetate transformation (Schiestl and Gietz, 1989, Current Genetics **16**:339-346) of 5 µg Not I linearized pRSN303.A/F9HR/340 or pRSN303.A/F9HR/34 into the haploid *Saccharomyces cerevisiae* strain YPH599/HYA32G5. This yeast clone contains a 650 kb YAC encoding the human factor IX gene (Campbell *et al*., 1991, Proc. Natl. Acad. Sci. USA **88**:5744-5748). Transformants were selected on minimal plates without histidine and colony purified, and tested for the ability to grow in the absence of uracil.

His+ transformants were replicated onto minimal plates lacking uracil and only his+/ura+ colonies were screened by PCR to verify homologous recombination at the Factor IX locus. Two primers were synthesized: primer g was located within the genomic sequence 5' of the 340 bp 5' Factor IX homology and primer h corresponded to a region in the targeting plasmid between the 340 bp 5' Factor IX homology and the HIS3 gene. A homologous recombination event would generate a novel amplification product of 422 bp. Transformants were pooled in groups of three after growth of individual colonies overnight in YPDA media. The pooled cells were spheroplasted with zymolyase (Philippsen *et al.,* 1991, Methods in Enzymology **194**:169-174), lysed by boiling for 10 minutes and a 10 µl aliquot of each sample used for PCR analysis. PCR reactions of 50 µl contained 10 mM Tris-HCl (pH 9.0 at 25°C), 50 mM KCl, 1.5 mM MgCl₂, 0.01% gelatin, 0.1% Triton X-100, 200 mM dNTPs, 1 mM each of the primers described above and 1.5 U of Taq DNA polymerase (Promega). Following an initial incubation at 94°C for 3 minutes, the samples were subjected to 40 cycles of denaturation at 94°C for 1 minute, annealing at 55°C for 2 minutes and extension at 72°C for 3 minutes. At the end of the 40 cycles, the samples were incubated an additional 5 minutes at 72°C. 5 µl of each sample was analyzed on a 1% agarose gel and stained with ethidium bromide. Almost all of the lysates analyzed contained the correct size fragment. PCR analysis using purified genomic DNA isolated from individual colonies derived from the positive pools demonstrated that all of the samples tested produced the correct 422 bp amplification product characteristic of the targeted YAC.

### Southern Analysis of Targeted Yeast DNA

Southern analysis of some of the PCR positive clones was used to further characterize the targeted events. Correct targeting of the Factor IX locus would result in a mobility shift of a 1.4 kb Xba I fragment found in the unmodified YAC to 9.7 kb due to the 8.3 kb insertion within the first exon of Factor IX, His3, pRS, MClneo, SV2DHFR and CMV IE. The expected 9.7 kb fragment was detected from most of the PCR positive transformants using end labeled PCR primer g as a probe, compared to the 1.4 kb fragment detected in the unmodified yeast YPH599/HYA32G5.

Southern analysis was also carried out using EcoRI digested genomic DNA with two different probes. When used as a probe, the 340 bp 5' Factor IX fragment from the targeting plasmid detects 12.8 kb EcoRI fragment in the unmodified YAC. This fragment was absent in targeted clones and replaced by a 9.0 kb fragment, due to the insertion by targeting of an additional EcoRI site at the 5' end of MClneo. The 9.0 kb EcoRI fragment was also detected in the targeted YAC DNA, as well as a 6.1 kb EcoRI fragment internal to the targeting plasmid, using a probe which spans the entire neo gene.

Transfer of the activated Factor IX gene to CHO and analysis of Expression: Yeast genomic DNA was prepared from the targeted Factor IX YAC and used to transfect CHO DHFR-DUKX B1 cells (Urlaub and Chasin, 1980, PNAS 7:4216-4220). Genomic DNA was extracted from the targeted YAC-pRSN303.A/F9HR/34 clones by spheroplasting 5x10¹⁰ yeast cells as described by Philippsen *et al.*, (Philippsen *et al*., 1991, Methods in Enzymology, **194**:169-174), followed by lysis in 20 ml of 0.5% SDS, 10 mM Tris pH 7.5, 2 mM EDTA and 0.5 mg/ml Proteinase K and incubation at 50°C for 2-3 hours. The lysate was phenol extracted, chloroform extracted, adjusted to 100 mM NaCl and genomic DNA precipitated with an equal volume of isopropanol. The DNA was then resuspended in 10 mM Tris pH 7.5, 1 mM EDTA, adjusted to 20 µg/ml RNAse A and digested for 30' at 37°C. Following RNAse treatment, the DNA was phenol extracted, chloroform extracted, adjusted to 100 mM NaCl and precipitated with an equal volume of isopropanol. Genomic DNA, resuspended in 5 ml of 10 mM Tris pH 7.5, 1 mM EDTA was dialyzed against 3 changes of 4 liters 10 mM Tris pH 7.5, 1 mM EDTA, for 48 hours, followed by concentration by isopropanol precipitation as described above and resuspended at a final concentration of 0.5 µg/µl in 10 mM Tris pH 7.5, 1 mM EDTA.

For transfection, 1.5x10⁶ CHO DHFR-cells were plated 12 hr prior to use on 10 cm dishes in DME/F12 media supplemented with 10% fetal bovine serum, glycine, hypoxanthine and thymidine (nonselective media). The media was changed 2 hours prior to transfection to DME, 4.5 g/l glucose, 10% fetal bovine serum. 30 µg of yeast genomic DNA was diluted to 0.5 ml with sterile water in a 4 ml polystyrene tube. 0.5 ml of 0.5 M CaCl2 was added to the genomic DNA, gently mixed, followed immediately by addition of 1.0 ml of HNP (49 mM Hepes, pH 7.10, 274 mM NaCl, 1.4 mM Na₂HPO₄). 2 ml of mix was added to a 10 cm dish and the cells were incubated at 37°C. Four hours later, the calcium phosphate precipitate was removed and the cells were incubated with 15% glycerol in HeBS (140 mM Nacl, 25 mM Hepes pH 7.1, 0.75 mM Na₂HPO₄) for 2.5 minutes at 37°C, washed twice with serum-free media, followed by growth in nonselective media for 48 hours. Fifty percent of the cells from each transfection were split 1:10 into DME/F12, 10% dFBS (dialyzed fetal bovine serum), glycine, thymidine and hypoxanthine and 400 µg/ml G418. The remaining cells were split into DME (4.5 g/l glucose), 10% dFBS and nonessential amino acids for selection of DHFR positive colonies. The media was changed every 3 days and surviving clones were picked into 24 well plates between days 10-16. Clones were expanded to 6 cm plates for ELISA media collection, then carried to 10 cm dishes for cryopreservation and genomic DNA preparation.

Seven transfections yielded a total of 24 G418 resistant colonies and 86 DHFR positive colonies. These were screened for secreted Factor IX using a commercially available ELISA kit (Asserachrom IX:Ag, #0410) and 45% were positive for secreted, immunoreactive protein. Although the average expression level was 2.35 ng Factor IX secreted/ml/10° cells/24 hours, Factor IX secreted at levels as high as 6 ng/ml/106 cells/24 hours was detected in 2 of the 51 positives (Table 1).

Analysis of Factor IX by immunoprecipitation and polyacrylamide gel electrophoresis confirmed that polypeptides of the correct size were synthesized and secreted. Confluent 6 cm dishes of 4 ELISA positive clones and one nonexpressing clone were pulse labeled with ³⁵S Cysteine for 6 hours, the supernatants harvested and cell lysates prepared. Labeled lysates or supernatants were incubated overnight with normal rabbit serum or Factor IX polyclonal antisera overnight, harvested with pansorbin, washed in detergent buffer, boiled and run under reducing conditions on a 10% SDS polyacrylamide gel as described by Smith *et al*. (Smith *et al.,* 1987). Two polypeptides were immunoprecipitated only in cell lysates derived from clones that expressed immunoreactive Factor IX. The major species, a 56 kd polypeptide, corresponds to the primary translation product of Factor IX mRNA. The minor 67 kd polypeptide probably corresponds to a partially glycosylated or γ-carboxylated form of Factor IX. Factor IX antisera precipitated a single 72 kd polypeptide from conditioned media of these clones, the species believed to be the biologically active form of Factor IX (Miletich *et al*., 1980, Anal. Biochem. 304-310).

Southern blot analysis was carried out on 21 Factor IX ELISA positive clones (both neo' and DHFR positive) and one DHFR positive, Factor IX ELISA negative clone to confirm that the complete Factor IX gene was transfered. Eco RI digests of genomic DNA were probed with the 865 bp Apa I/Eco RI fragment derived from the 3' noncoding region of exon 8. The 3' end of this EcoRI fragment is located 32.5 kb from the transcription initiation site of the Factor IX gene. All of the positive clones contained the expected 5.5 kb band, identical to HT1080 DNA and absent from untransfected CHO cells. Therefore, DHFR positive Factor IX expressing CHO clones have successfully transferred at least 35.5 kb and G418 resistant clones have transferred at least 39 kb of genomic DNA.

**Table I**

| **Factor IX Positive Clones** | | | | |
|---|---|---|---|---|
| **DNA #** | **CLONE #** | **Cellsx10^6** | **ngFIX/ml** | **ng FIX/ml/10^6 cells** |
| 97 neo | 9.5 | 29.2 | 20.33 | 0.70 |
| 95 neo | 2.1 | 9.6 | 17.01 | 1.77 |
| 26 neo | 5.21 | 31 | 70.54 | 2.28 |
| 47 | 2.22 | 9.9 | 26.22 | 2.65 |
| 43 | 3.32 | 10.7 | 69.17 | 6.46 |

It is evident from the above results, that a simple accurate technique has been developed which allows for the ready manipulation of genes with high efficiency, introduction of amplifiable markers to allow for amplification of a target gene, modifications of genes, and transfer of the resulting modified chromosomal DNA to a mammalian expression host, to provide for efficient expression of the desired product having the same, substantially the same, or different composition from the natural product. High protein yields are attainable as compared to other methods of protein production, *e.g.* the use of cDNA expression vectors. Thus, one can obtain processing, combinations of products due to variation in splicing, processing, such as glycosylation, acetylation, methylation, or the like, as well as high and efficient levels of stable production of the desired product. Thus, a rapid, efficient methodology is provided for producing expression constructs for transformation into mammalian expression hosts, without the need for isolating and purifying the target gene or cDNA and allowing for modification of the target gene with high efficiency.

## Claims

1. A yeast artificial chromosome having an expressible target gene, comprising:
a target region, said target region comprising a mammalian target gene and a transcriptional regulatory element in operative association with said target gene,
wherein said transcriptional regulatory element is heterologous to said target gene, and wherein said transcriptional regulatory element is capable of directing expression of said target gene in a mammalian host cell.

2. A yeast artificial chromosome as claimed in Claim 1, wherein said target gene contains at least one mutation.

3. A yeast artificial chromosome as claimed in Claim 1 or Claim 2 further comprising an amplifiable gene operatively associated with said target region, wherein said target region is amplifiable in the mammalian expression host cell when said amplifiable gene is amplified by the appropriate selection agent.

4. A yeast artificial chromosome as claimed in Claim 3, wherein said amplifiable gene is selected from the group consisting of dihydrofolate reductase, metallothionein-I, metallothionein-II, adenosine deaminase, ornithine decarboxylase, and glutamine synthetase.

5. A yeast artificial chromosome as claimed in any one of Claims 1 to 4 in which said mammalian gene is a human gene.

6. A yeast artificial chromosome as claimed in any one of Claims 1 to 5 in which said transcriptional regulatory element is a promoter and/or enhancer.

7. A yeast artificial chromosome as claimed in Claim 6 in which said promoter and/or enhancer is a human cytomegalovirus immediate early region promoter/enhancer.

8. A yeast cell having the yeast artificial chromosome as claimed in any one of Claims 1 to 4.

9. A mammalian expression host cell transformed with a yeast artificial chromosome as claimed in Claim 1, or non-human animal progeny of the transformed host cell, that express said target gene controlled by said transcriptional regulatory element.

10. A mammalian expression host cell transformed with a yeast artificial chromosome as claimed in Claim 3 or Claim 4, or progeny of the transformed host cell, that express said target gene controlled by said transcriptional regulatory element, which said target region is amplified under conditions that amplify the amplifiable gene.

11. A mammalian expression host cell as claimed in Claim 9 or Claim 10, wherein said mammalian expression host cell is a Chinese hamster ovary cell, a monkey kidney cell, a C127 mouse fibroblast cell, a 3T3 mouse cell, or a Vero cell.

12. A mammalian expression host cell as claimed in any one of Claims 9 to 11 in which said target gene is a human gene.

13. A mammalian expression host cell as claimed in any one of Claims 9 to 12 in which said transcriptional regulatory element is a promoter and/or enhancer.

14. A mammalian continuous cell line as claimed in Claim 13 in which the promoter and/or enhancer is a human cytomegalovirus immediate early region promoter/enhancer.

15. A method for producing a yeast artificial chromosome having an expressible target gene, comprising:
(a) integrating a nucleic acid via homologous recombination into a yeast artificial chromosome having a target gene, wherein the nucleic acid comprises a heterologous transcriptional regulatory element and a nucleotide region homologous to a region of at least 50 nucleotides of the yeast artificial chromosome, and
(b) selecting a yeast artificial chromosome in which expression of the target gene is regulable in a mammalian host cell by the heterologous transcriptional regulatory element.

16. A method for producing a yeast artificial chromosome having an amplifiable target gene, comprising:
(a) integrating a nucleic acid via homologous recombination into a yeast artificial chromosome having a target gene, wherein the nucleic acid comprises a transcriptional regulatory element, an amplifiable gene, and a nucleotide region homologous to a region of at least 50 nucleotides of the yeast artificial chromosome, and
(b) selecting a yeast artificial chromosome in which the target gene is both regulable by the integrated transcriptional regulatory element and amplifiable in a mammalian host cell when the amplifiable gene is amplified by the appropriate selection agent.

17. A method as claimed in Claim 16 wherein the amplifiable gene is selected from the group consisting of dihydrofolate reductase, metallothionein-I, metallothionein-II, adenosine deaminase, ornithine decarboxylase, and glutamine synthetase.

18. A method as claimed in Claim 16 or Claim 17 wherein integration of the nucleic acid introduces at least one mutation into the target gene.

19. A method for producing a mammalian expression host cell for use in protein production in culture, comprising:
(a) integrating, via targeted homologous recombination, a transcriptional regulatory element heterologous to a mammalian target gene contained in a yeast artificial chromosome maintained in a yeast host cell, so that the integrated transcriptional regulatory element is operably associated with the mammalian target gene to form a recombined mammalian target gene; and
(b) transferring the recombined mammalian target gene into a mammalian host cell capable of expressing the target gene product under the control of the transcriptional regulatory element, so that the mammalian target gene product is expressed by the mammalian host cell in culture.

20. A method for producing a mammalian host cell as claimed in Claim 19, which further comprises integrating, via targeted homologous recombination, an amplifiable gene proximal to the mammalian target gene contained in the yeast artificial chromosome maintained in the yeast host cell, so that the coding sequence of the mammalian target gene product is not disrupted, and so that when the recombined mammalian target gene is transferred into the mammalian host cell, the mammalian target gene controlled by the heterologous transcriptional regulatory element is amplified when the mammalian host cell is cultured under conditions that amplify the amplifiable gene.

21. A method for production of a mammalian gene product in cell culture, comprising culturing a mammalian host cell which expresses an exogenous mammalian target gene controlled by a transcriptional regulatory element heterologous to the mammalian target gene, and recovering the mammalian target gene product from the cell culture, in which the mammalian host cell was prepared by:
(a) integrating, via targeted homologous recombination, the transcriptional regulatory element heterologous to the mammalian target gene into the target gene contained in a yeast artificial chromosome maintained in a yeast host cell, so that the integrated transcriptional regulatory element is operably associated with the mammalian target gene contained in the yeast artificial chromosome to form a recombined mammalian target gene; in the yeast host cell and
(b) transferring the recombined mammalian target gene to a mammalian host cell capable of expressing the target gene product under the control of the integrated transcriptional regulatory element so that the mammalian target gene product is expressed by the mammalian host cell in culture.

22. A method as claimed in Claim 21 further comprising integrating, via targeted homologous recombination, an amplifiable gene proximal to the mammalian target gene contained in the yeast artificial chromosome maintained in the yeast host cell, so that the coding sequence of the mammalian target gene product is not disrupted and so that when the recombined mammalian target gene is transferred into the mammalian host cell, the mammalian target gene controlled by the heterologous transcriptional regulatory element is amplified when the mammalian host cell is cultured under conditions that amplify the amplifiable gene.

23. A method as claimed in Claim 22 in which the amplifiable gene is dihydrofolate reductase, metallothionein-I, metallothionein-II, adenosine deaminase, ornithine decarboxylase, or glutamine synthetase.

24. A method as claimed in Claim 22 or Claim 23 in which the mammalian target gene is a human gene.

25. A method as claimed in any one of Claims 22 to 24 in which the transcriptional regulatory element is a promoter and/or enhancer.

26. A method as claimed in Claim 25 in which the promoter and/or enhancer is a human cytomegalovirus immediate early region promoter/enhancer.

27. A method as claimed in any one of Claims 22 to 26 in which the mammalian host cell is a Chinese hamster ovary cell, a monkey kidney cell, a C127 mouse fibroblast cell, a 3T3 mouse cell, or a Vero cell.

28. A method for activating a target gene, comprising:
integrating a transcriptional regulatory element into a yeast artificial chromosome comprising the gene so that the transcriptional regulatory element is heterologous to, and operatively associated with the gene and so that the transcriptional regulatory element activates the gene in an appropriate host cell.

## Patentansprüche

1. Künstliches Hefechromosom mit einem ausprägungsfähigen Zielgen, darin inbegriffen ein Zielbereich, der ein Zielgen eines Säugers und im operativen Zusammenwirken mit dem Zielgen ein Regulationselement für die Transkription beinhaltet,
wobei das Regulationselement für die Transkription heterolog zum Zielgen und fähig zu einer gerichteten Ausprägung des Zielgens in einer Wirtszelle eines Säugers ist.

2. Künstliches Hefechromosom nach Anspruch 1, wobei das Zielgen mindestens eine Mutation enthält.

3. Künstliches Hefechromosom nach Anspruch 1 oder 2, das ferner ein amplifizierbares Gen im Wirkungszusammenhang mit dem Zielbereich aufweist, wobei der Zielbereich in der Ausprägungs-Wirtszelle des Säugers amplifizierbar ist, wenn das amplifizierbare Gen durch den geeigneten Selektionswirkstoff amplifiziert wird.

4. Künstliches Hefechromosom nach Anspruch 3, wobei das amplifizierbare Gen aus der Gruppe gewählt wird, die aus Dihydrofolatreductase, Metallothionein-I, Metallothionein-II, Adenosindesaminase, Ornithin-Decarboxylase und Glutaminsynthetase besteht.

5. Künstliches Hefechromosom nach einem der Ansprüche 1 bis 4, wobei das Säugergen ein Gen des Menschen ist.

6. Künstliches Hefechromosom nach einem der Ansprüche 1 bis 5, wobei das Regulationselement für die Transkription ein Promotor und/oder Verstärker ist.

7. Künstliches Hefechromosom nach Anspruch 6, wobei der Promotor und/oder Verstärker von der Art ist, die beim Zytomegalie-Virus des Menschen im unmittelbaren frühen Bereich wirkt.

8. Hefezelle, die ein künstliches Hefechromosom nach einem der Ansprüche 1 bis 4 enthält.

9. Mit künstlichem Hefechromosom nach Anspruch 1 transformierte ausprägungsfähige Säuger-Wirtszelle oder nichtmenschliches, tierisches Nachkommengenom der transformierten Wirtszelle, welches das vom Regulationselement für die Transkription gesteuerte Zielgen ausprägt.

10. Mit künstlichem Hefechromosom nach Anspruch 3 oder 4 transformierte ausprägungsfähige Säuger-Wirtszelle oder Nachkommengenom der transformierten Wirtszelle, welches das vom Regulationselement für die Transkription gesteuerte Zielgen ausprägt, wobei der Zielbereich unter Bedingungen amplifiziert wird, die das amplifizierbare Gen amplifizieren.

11. Ausprägungsfähige Säuger-Wirtszelle nach Anspruch 9 oder 10, wobei die ausprägungsfähige Säuger-Wirtszelle eine Eierstockzelle eines chinesischen Hamsters, eine Affennierenzelle, eine Fibroblastenzelle der Maus C127, eine Zelle der Maus 3T3 oder eine Verozelle ist.

12. Ausprägungsfähige Säuger-Wirtszelle nach einem der Ansprüche 9 bis 11, wobei das Zielgen ein Gen des Menschen ist.

13. Ausprägungsfähige Säuger-Wirtszelle nach einem der Ansprüche 9 bis 12, wobei das Regulationselement für die Transkription ein Promotor und/-oder Verstärker ist.

14. Kontinuierliche Säuger-Zell-Linie nach Anspruch 13, wobei der Promotor und/oder Verstärker von der Art ist, die beim Zytomegalie-Virus des Menschen im unmittelbaren frühen Bereich wirkt.

15. Verfahren zur Herstellung eines künstlichen Hefechromosoms mit einem ausprägungsfähigen Zielgen, beinhaltend
(a) den Einbau einer Nucleinsäure durch homologe Rekombination in einem künstlichen Hefechromosom, das ein Zielgen enthält, wobei die Nucleinsäure ein heterologes Regulationselement für die Transkription und einen Nucleotidbereich enthält, der homolog zu einem Bereich von mindestens 50 Nucleotiden des künstlichen Hefechromosoms ist und
(b) die Wahl eines künstlichen Hefechromosoms, in welchem die Ausprägung des Zielgens in einer Säuger-Wirtszelle durch das heterologe Regulationselement für die Transkription regulierbar ist.

16. Verfahren zur Herstellung eines künstlichen Hefechromosoms, das ein amplifizierbares Zielgen aufweist, beinhaltend
(a) den Einbau einer Nucleinsäure durch homologe Rekombination in einem künstlichen Hefechromosom, das ein Zielgen enthält, wobei die Nucleinsäure ein Regulationselement für die Transkription, ein amplifizierbares Gen und einen Nucleotidbereich enthält, der homolog zu einem Bereich von mindestens 50 Nucleotiden des künstlichen Hefechromosoms ist und
(b) die Wahl eines künstlichen Hefechromosoms, in welchem das Zielgen sowohl durch das integrierte Regulationselement für die Transkription regulierbar, als auch in der Säuger-Wirtszelle amplifizierbar ist, wenn das amplifizierbare Gen durch den geeigneten Selektionswirkstoff amplifiziert wird.

17. Verfahren nach Anspruch 16, wobei das amplifizierbare Gen aus der Gruppe gewählt wird, die aus Dihydrofolatreductase, Metallothionein-I, Metallothionein-II, Adenosindesaminase, Ornithin-Decarboxylase und Glutaminsynthetase besteht.

18. Verfahren nach Anspruch 16 oder 17, wobei der Einbau der Nucleinsäure mindestens eine Mutation in das Zielgen einführt.

19. Verfahren zur Herstellung einer ausprägungsfähigen Säuger-Wirtszelle zur Verwendung bei der Proteinherstellung in Kulturen, beinhaltend
(a) die Integration durch gezielte homologe Rekombination eines heterologen Regulationselementes für die Transkription, in das Zielgen eines Säugers, das in dem in einer Hefe-Wirtszelle gehaltenen künstlichen Hefechromosom enthalten ist, so dass das integrierte Regulationselement für die Transkription im Wirkungszusammenhang mit dem Säuger-Zielgen ein rekombiniertes Säuger-Zielgen bilden kann und
(b) die Übertragung des rekombinierten Säuger-Zielgens in eine Säuger-Wirtszelle, die in der Lage ist, das Zielgen-Produkt vom Regulationselement für die Transkription gesteuert so auszuprägen, dass das Produkt des Säuger-Zielgens durch die Säuger-Wirtszelle in der Kultur ausgeprägt ist.

20. Verfahren zur Herstellung einer Säuger-Wirtszelle nach Anspruch 19, ferner beinhaltend die Integration durch gezielte homologe Rekombination, eines amplifizierbaren Gens proximal zum Säuger-Zielgen, das in dem in einer Hefe-Wirtszelle gehaltenen künstlichen Hefechromosom enthalten ist, so, dass die Codierungssequenz des Säuger-Zielgenproduktes nicht abgebrochen wird und so, dass bei Übertragung des rekombinierten Säuger-Zielgens in die Säuger-Wirtszelle das vom heterologen Regulationselement für die Transkription gesteuerte Säuger-Zielgen amplifiziert wird, wenn die Säuger-Wirtszelle unter Bedingungen kultiviert wird, die das amplifizierbare Gen amplifizieren.

21. Verfahren zur Herstellung eines Säugergenproduktes in Zellkultur, beinhaltend die Kultivierung einer Säuger-Wirtszelle, die ein exogenes Säuger-Zielgen ausprägt, das von einem für das Säuger-Zielgen heterologen Regulationselement für die Transkription gesteuert wird und Rückgewinnung des Säuger-Zielgenproduktes aus der Zellkultur, in der die Säuger-Wirtszelle gewonnen wurde, durch
(a) Integration durch gezielte homologe Rekombination eines heterologen Regulationselementes für die Transkription, in das Zielgen eines Säugers, das in dem in einer Hefe-Wirtszelle gehaltenen künstlichen Hefechromosom enthalten ist, so dass das integrierte Regulationselement für die Transkription im Wirkungszusammenhang mit dem Säuger-Zielgen, das im künstlichen Hefechromosom enthalten ist, ein rekombiniertes Säuger-Zielgen in der Hefe-Wirtszelle bilden kann und
(b) die Übertragung des rekombinierten Säuger-Zielgens in eine Säuger-Wirtszelle, die in der Lage ist, das Zielgen-Produkt vom Regulationselement für die Transkription gesteuert so auszuprägen, dass das Produkt des Säuger-Zielgens durch die Säuger-Wirtszelle in der Kultur ausgeprägt ist.

22. Verfahren nach Anspruch 21, ferner beinhaltend die Integration durch gezielte homologe Rekombination, eines amplifizierbaren Gens proximal zum Säuger-Zielgen, das in dem in einer Hefe-Wirtszelle gehaltenen künstlichen Hefechromosom enthalten ist, so, dass die Codierungssequenz des Säuger-Zielgenproduktes nicht abgebrochen wird und so, dass bei Übertragung des rekombinierten Säuger-Zielgens in die Säuger-Wirtszelle das vom heterologen Regulationselement für die Transkription gesteuerte Säuger-Zielgen amplifiziert wird, wenn die Säuger-Wirtszelle unter Bedingungen kultiviert wird, die das amplifizierbare Gen amplifizieren.

23. Verfahren nach Anspruch 22, wobei das amplifizierbare Gen Dihydrofolatreductase, Metallothionein-I, Metallothionein-II, Adenosindesaminase, Ornithin-Decarboxylase und Glutaminsynthetase ist.

24. Verfahren nach Anspruch 22 oder 23, wobei das Säuger-Zielgen ein Gen des Menschen ist.

25. Verfahren nach einem der Ansprüche 22 bis 24, wobei das Regulationselement für die Transkription ein Promotor und/-oder Verstärker ist.

26. Verfahren nach Anspruch 25, wobei der Promotor und/oder Verstärker von der Art ist, die beim Zytomegalie-Virus des Menschen im unmittelbaren frühen Bereich wirkt.

27. Verfahren nach einem der Ansprüche 22 bis 26, wobei die Säuger-Wirtszelle eine Eierstockzelle eines chinesischen Hamsters, eine Affennierenzelle, eine Fibroblastenzelle der Maus C127, eine Zelle der Maus 3T3 oder eine Verozelle ist.

28. Verfahren zur Aktivierung eines Zielgens, beinhaltend die Integration eines Regulationselementes für die Transkription in ein künstliches Hefechromosom, welches das Gen enthält, so, dass das Regulationselement für die Transkription heterolog ist und im Wirkungszusammenhang mit dem Gen steht und das Gen in einer geeigneten Wirtszelle aktiviert.

## Revendications

1. Chromosome artificiel de levure ayant un gène cible exprimable, comprenant :
une région cible, ladite région cible comprenant un gène cible de mammifère et un élément régulateur de transcription en association opérante avec ledit gène cible,
dans lequel ledit élément régulateur de transcription est hétérologue audit gène cible, et dans lequel ledit élément régulateur de transcription est capable de diriger l'expression dudit gène cible dans une cellule hôte de mammifère.

2. Chromosome artificiel de levure tel que revendiqué dans la revendication 1, dans lequel ledit gène cible contient au moins une mutation.

3. Chromosome artificiel de levure tel que revendiqué dans la revendication 1 ou la revendication 2, comprenant en outre un gène amplifiable associé de manière opérante à ladite région cible, dans lequel ladite région cible est amplifiable dans la cellule hôte d'expression de mammifère quand ledit gène amplifiable est amplifié par l'agent de sélection approprié.

4. Chromosome artificiel de levure tel que revendiqué dans la revendication 3, dans lequel ledit gène amplifiable est choisi dans le groupe constitué par la dihydrofolate-réductase, la métallothionéine-I, la métallothionéine -II, l'adénosine désaminase, l'ornithine décarboxylase, et la glutamine synthétase.

5. Chromosome artificiel de levure tel que revendiqué dans l'une quelconque des revendications 1 à 4, dans lequel ledit gène de mammifère est un gène humain.

6. Chromosome artificiel de levure tel que revendiqué dans l'une quelconque des revendications 1 à 5, dans lequel ledit élément régulateur de transcription est un promoteur et/ou un amplificateur.

7. Chromosome artificiel de levure tel que revendiqué dans la revendication 6, dans lequel ledit promoteur et/ou amplificateur est un promoteur/amplificateur de région très précoce de cytomégalovirus humain.

8. Cellule de levure ayant le chromosome artificiel de levure tel que revendiqué dans l'une quelconque des revendications 1 à 4.

9. Cellule hôte d'expression de mammifère transformée avec un chromosome artificiel de levure tel que revendiqué dans la revendication 1, ou descendance animale non humaine de la cellule hôte transformée, qui exprime ledit gène cible contrôlé par ledit élément régulateur de transcription.

10. Cellule hôte d'expression de mammifère transformée avec un chromosome artificiel de levure tel que revendiqué dans la revendication 3 ou la revendication 4, ou descendance de la cellule hôte transformée, qui exprime ledit gène cible contrôlé par ledit élément régulateur de transcription, ladite région cible étant amplifiée dans des conditions qui amplifient le gène amplifiable.

11. Cellule hôte d'expression de mammifère telle que revendiquée dans la revendication 9 ou la revendication 10, ladite cellule hôte d'expression de mammifère étant une cellule d'ovaire d'hamster chinois, une cellule de rein de singe, une cellule fibroblaste de souris C127, une cellule de souris 3T3, ou une cellule Vero.

12. Cellule hôte d'expression de mammifère telle que revendiquée dans l'une quelconque des revendications 9 à 11, dans laquelle ledit gène cible est un gène humain.

13. Cellule hôte d'expression de mammifère telle que revendiquée dans l'une quelconque des revendications 9 à 12, dans laquelle ledit élément régulateur de transcription est un promoteur et/ou un amplificateur.

14. Lignée cellulaire continue de mammifère telle que revendiquée dans la revendication 13, dans laquelle le promoteur et/ou l'amplificateur est un promoteur/amplificateur de région très précoce de cytomégalovirus humain.

15. Procédé de préparation d'un chromosome artificiel de levure ayant un gène cible exprimable, comprenant les étapes consistant à :
(a) intégrer un acide nucléique via une recombinaison homologue dans un chromosome artificiel de levure ayant un gène cible, dans lequel l'acide nucléique comprend un élément régulateur de transcription hétérologue et une région nucléotidique homologue à une région d'au moins 50 nucléotides du chromosome artificiel de levure, et
(b) choisir un chromosome artificiel de levure dans lequel l'expression du gène cible est régulable dans une cellule hôte de mammifère par l'élément régulateur de transcription hétérologue.

16. Procédé de préparation d'un chromosome artificiel de levure ayant un gène cible amplifiable, comprenant les étapes consistant à :
(a) intégrer un acide nucléique via une recombinaison homologue dans un chromosome artificiel de levure ayant un gène cible, dans lequel l'acide nucléique comprend un élément régulateur de transcription, un gène amplifiable, et une région nucléotidique homologue à une région d'au moins 50 nucléotides du chromosome artificiel de levure, et
(b) choisir un chromosome artificiel de levure dans lequel le gène cible est à la fois régulable par l'élément régulateur de transcription intégré et amplifiable dans une cellule hôte de mammifère quand le gène amplifiable est amplifié par un agent de sélection approprié.

17. Procédé tel que revendiqué dans la revendication 16, dans lequel ledit gène amplifiable est choisi dans le groupe constitué par la dihydrofolate-réductase, la métallothionéine-I, la métallothionéine-II, l'adénosine désaminase, l'ornithine décarboxylase, et la glutamine synthétase.

18. Procédé tel que revendiqué dans la revendication 16 ou la revendication 17, dans lequel l'intégration de l'acide nucléique introduit au moins une mutation dans le gène cible.

19. Procédé de préparation d'une cellule hôte d'expression de mammifère pour l'utilisation dans une préparation de protéine par culture, comprenant les étapes consistant à :
(a) intégrer, via une recombinaison homologue ciblée, un élément régulateur de transcription hétérologue à un gène cible de mammifère contenu dans un chromosome artificiel de levure maintenu dans une cellule hôte de levure, de manière que l'élément régulateur de transcription intégré soit associé de manière opérante au gène cible de mammifère pour former un gène cible de mammifère recombiné ; et
(b) transférer le gène cible de mammifère recombiné dans une cellule hôte de mammifère capable d'exprimer le produit de gène cible sous le contrôle de l'élément régulateur de transcription, de manière que le produit de gène cible de mammifère soit exprimé par la cellule hôte de mammifère dans la culture.

20. Procédé de préparation d'une cellule hôte de mammifère tel que revendiqué dans la revendication 19, qui comprend en outre l'étape consistant à intégrer, via une recombinaison homologue ciblée, un gène amplifiable proximal au gène cible de mammifère contenu dans le chromosome artificiel de levure maintenu dans la cellule hôte de levure, de manière que la séquence codante du produit de gène cible de mammifère ne soit pas interrompue, et de manière que, lorsque le gène cible de mammifère recombiné est transféré dans la cellule hôte de mammifère, le gène cible de mammifère contrôlé par l'élément régulateur de transcription hétérologue soit amplifié quand la cellule hôte de mammifère est cultivée dans des conditions qui amplifient le gène amplifiable.

21. Procédé de préparation d'un produit de gène de mammifère dans une culture cellulaire, comprenant la culture d'une cellule hôte de mammifère qui exprime un gène cible de mammifère exogène contrôlé par un élément régulateur de transcription hétérologue au gène cible de mammifère, et la récupération du produit de gène cible de mammifère à partir de la culture cellulaire, dans lequel on prépare la cellule hôte de mammifère par les étapes consistant à :
(a) intégrer, via une recombinaison homologue ciblée, l'élément régulateur de transcription hétérologue au gène cible de mammifère dans le gène cible contenu dans un chromosome artificiel de levure maintenu dans une cellule hôte de levure, de manière que l'élément régulateur de transcription intégré soit associé de manière opérante au gène cible de mammifère contenu dans le chromosome artificiel de levure pour former un gène cible de mammifère recombiné ; dans la cellule hôte de levure, et
(b) transférer le gène cible de mammifère recombiné à une cellule hôte de mammifère capable d'exprimer le produit de gène cible sous le contrôle de l'élément régulateur de transcription intégré, de manière que le produit de gène cible de mammifère soit exprimé par la cellule hôte de mammifère dans la culture.

22. Procédé tel que revendiqué dans la revendication 21, comprenant en outre l'intégration, via une recombinaison homologue ciblée, d'un gène amplifiable proximal au gène cible de mammifère contenu dans le chromosome artificiel de levure maintenu dans la cellule hôte de levure, de manière que la séquence codante du produit de gène cible de mammifère ne soit pas interrompue et de manière que, quand le gène cible de mammifère recombiné est transféré dans la cellule hôte de mammifère, le gène cible de mammifère contrôlé par l'élément régulateur de transcription hétérologue soit amplifié quand la cellule hôte de mammifère est cultivée dans des conditions qui amplifient le gène amplifiable.

23. Procédé tel que revendiqué dans la revendication 22, dans lequel le gène amplifiable est la dihydrofolate-réductase, la métallothionéine-I, la métallothionéine-II, l'adénosine désaminase, l'ornithine décarboxylase, ou la glutamine synthétase.

24. Procédé tel que revendiqué dans la revendication 22 ou la revendication 23, dans lequel le gène cible de mammifère est un gène humain.

25. Procédé tel que revendiqué dans l'une quelconque des revendications 22 à 24, dans lequel l'élément régulateur de transcription est un promoteur et/ou un amplificateur.

26. Procédé tel que revendiqué dans la revendication 25, dans lequel le promoteur et/ou l'amplificateur est un promoteur/amplificateur de région très précoce de cytomégalovirus humain.

27. Procédé tel que revendiqué dans l'une quelconque des revendications 22 à 26, dans lequel la cellule hôte d'expression de mammifère est une cellule d'ovaire d'hamster chinois, un cellule le rein de singe, une cellule fibroblaste de souris C127, une cellule de souris 3T3, ou une cellule Vero.

28. Procédé pour activer un gène cible, comprenant :
l'intégration d'un élément régulateur de transcription dans un chromosome artificiel de levure comprenant le gène de manière que l'élément régulateur de transcription soit hétérologue, et associé de manière opérante au gène et de manière que l'élément régulateur de transcription active le gène dans une cellule hôte appropriée.
